# EUROPEAN PATENT SPECIFICATION

(11) **EP 4 405 331 B1**
(45) Date of publication and mention of the grant of the patent: **11.11.2026**
(21) Application number: 22793684.6
(22) Date of filing: 19.09.2022
(51) Int. Cl.: C07C 263/10, C07C 265/14, C07C 209/36, C07C 211/50, C07C 201/08, C07C 205/06

(54) **A PROCESS FOR PREPARING AT LEAST ONE AROMATIC ISOCYANATE**
VERFAHREN ZUR HERSTELLUNG VON MINDESTENS EINEM AROMATISCHEN ISOCYANAT
PROCÉDÉ DE PRÉPARATION D'AU MOINS UN ISOCYANATE AROMATIQUE

(30) Priority: 20.09.2021 EP 21197762
(43) Date of publication of application: 31.07.2024
(73) Proprietor: BASF SE, 67056 Ludwigshafen am Rhein (DE)
(72) Inventor: MATTERN, Jonas, 01986 Schwarzheide (DE); BUETTNER, Johannes, 01986 Schwarzheide (DE); FRITZ, Ruediger, 02994 Bernsdorf (DE); KOELLER, Maximilian, 16244 Schorfheide (DE); JOSCH, Jan Pablo, 67056 Ludwigshafen am Rhein (DE)
(74) Representative: BASF IP Association
(86) International application number: PCT/EP2022/075965
(87) International publication number: WO 2023/041781

(56) References cited:
- US-A1- 2017 152 210
- US-A1- 2018 290 968

## Description

The present invention relates to a process for preparing at least one aromatic isocyanate comprising a specific starting-up, and to a chemical unit U for carrying out said process.

Aromatic isocyanates are often prepared via a continuous or discontinuous process in suitable plant sections or plants. Since such a process comprises several sub-processes, a plant section or plant comprises one or more chemical processing sub-units for processing the sub-processes. It is also possible to carry out a sub-process in parallel in several chemical processing sub-units. Considering that preparing an aromatic isocyanate usually includes nitration, hydrogenation and phosgenation of an aromatic compound, as for example in the preparation of toluene diisocyanate starting from toluene, a process for preparing at least one aromatic isocyanate includes several sub-processes wherein a sub-process is carried out in a chemical processing sub-unit. The product compositions obtained from the chemical processing units are then fed to further processing in subsequent chemical processing sub-units until a desired product composition is obtained.

During a regular operating mode of a sub-unit, the sub-process being carried out in said sub-unit thus comprises feeding an educt composition comprising at least one aromatic compound into said sub-unit and processing said composition in said sub-unit for obtaining a product composition comprising at least one aromatic compound, such that eventually at least one aromatic isocyanate is obtained from the latest chemical processing sub-unit. As used herein, an educt composition is equivalent to a reactant composition. Likewise the term educt, also spelled Edukt in the German language means reactant as defined by Baerns, M. et al in "Technische Chemie", Wiley, 2014, page 697. Therefore, as defined in the present invention, an educt composition is equivalent to a reactant composition.

In order to ensure a high performance of the one or more chemical processing units and chemical processing sub-units of a plant section or plant, regular maintenance, cleaning and optionally repairing of said chemical processing units or chemical processing sub-units is required.

For doing so, plant sections or plants comprising one or more chemical processing units and chemical processing sub-units have to be shut down in parts or completely. Parts of a plant section or plant not affected by maintenance, cleaning or repairing can stay in steady state, in particular if a return of a stream comprising a product composition or another circulating stream can be ensured. For instance, WO 2015/197522 A1 discloses a method in which plant sections not affected by maintenance, cleaning or repairing are operated in a so-called recirculation mode. It is disclosed therein that the product of such a plant section or of a further downstream plant section is fed into this plant section to maintain a circulating flow. In WO 2017/050776 A1 a similar method is disclosed in which plant sections not affected by maintenance, cleaning or repairing are operated under a circulating flow.

On the other hand, those parts of a plant section or plant which have been affected by maintenance have to be re-started. However, starting-up of a process carried out in one or more chemical processing units and chemical processing sub-units is an expensive procedure, in particular considering resource efficiency and energy costs. Resource efficiency relates to the influence of process parameters different to target process parameters on process performance. If for example an educt composition differs from a target educt composition having an optimized composition for further processing, an inferior conversion may be expected making recycling or wasting of excess starting material necessary. Energy costs include costs for heating or cooling of chemical processing units, chemical processing sub-units, separation devices, and other processing devices. Additionally, starting-up of a process can be time consuming. In the context of the present invention starting-up of a process is terminated in particular when the product composition meets the desired specifications, inter alia allowing further processing thereof in a subsequent process or process step.

Therefore, it was an object of the present invention to provide a novel process for preparing at least one aromatic isocyanate comprising a specific starting-up process. In particular, it was an object of the present invention to provide an improved process for preparing at least one aromatic isocyanate comprising a specific starting-up concerning resource efficiency.

Therefore, the present invention relates to a process for preparing at least one aromatic isocyanate, said process comprising n chemical sub-processes sp(i), wherein a sub-process sp(i) is carried out in a chemical processing sub-unit U(i), i=1...n, n≥1,
wherein during a regular operating mode of a sub-unit U(i), the sub-process sp(i) being carried out in said sub-unit U(i) comprises feeding an educt composition E(i) comprising at least one aromatic compound Z^{j}_{E}(i), j≥1, into said sub-unit U(i), processing said composition E(i) in said sub-unit U(i) and obtaining a product composition P(i) comprising at least one aromatic compound Z^{k}_{P}(i), k≥1,
wherein the chemical process comprises starting up said sub-process sp(i) in said sub-unit U(i),
wherein said starting-up comprises processing a chemical start-up composition S(i) in said sub-unit U(i), wherein said S(i) ≠ E(i) and wherein said S(i) comprises at least one of the aromatic compounds Z^{k}_{P}(i).

As mentioned above plant sections or plants comprising one or more chemical processing units and chemical processing sub-units have to be shut down in parts or completely if maintenance, cleaning or repairing needs to be carried out. Thus, it is preferred that the process according to the present invention further comprises, prior to starting up the sub-process sp(i) in the sub-unit U(i), shutting down the sub-process sp(i) in the sub-unit U(i) and keeping the sub-unit U(i) in a non-operation mode for a period of time Δt(i).

For starting up the sub-process sp(i) of the process according to the present invention, it is preferred that said starting up comprises feeding the chemical composition S(i) into the sub-unit U(i).

As defined above, the process according to the present invention comprises n chemical sub-processes sp(i), wherein a sub-process sp(i) is carried out in a chemical processing sub-unit U(i), i=1...n, n≥1. In the case where n=1 the process comprises only one sub-process being carried out in a single sub-unit which can be considered as a chemical unit U. In the case where n≥2, at least 2 sub-processes are comprised by the process of the present invention each being carried out in a chemical processing sub-unit. If n≥2, thus it n is at least 2, it is preferred that all sub-units U(i) are part of a chemical unit U.

In the case where all sub-units U(i) are part of a chemical unit U, it is preferred that U further comprises, in addition to the n chemical processing sub-units U(i), r storing devices D(m), m=1...r, r≥1. If the chemical unit U further comprises, in addition to the n chemical processing sub-units U(i), r storing devices D(m), m=1...r, r≥1, it is preferred that the process according to the present invention further comprises, during a regular operating mode of a sub-unit U(i), removing the product composition P(i) from said sub-unit U(i) and transferring a portion of said product composition P(i) removed from said sub-unit U(i) into a storing device D(m).

In the case where the product composition P(i) is removed from a sub-unit U(i) during a regular operating mode of said sub-unit U(i) and where a portion of said product composition P(i) removed from said sub-unit U(i) is transferred into a storing device D(m), it is preferred that, during starting up the sub-process sp(i), at least a portion of P(i), stored in D(m) during the regular operating mode of the sub-unit U(i), is fed as at least a portion of the chemical composition S(i) into the sub-unit U(i).

According to the present invention a chemical sub-process sp(i) is carried out in a chemical processing sub-unit U(i). Thus, a chemical processing sub-unit U(i) can be any conceivable means suitable for carrying out a chemical sub-process sp(i). It is preferred that the sub-unit U(i) is selected from the group consisting of a chemical reactor, a distillation column, a rectification column, a tank, a phase separator, a washing device, and a combination of two or more thereof.

According to the present invention the process for preparing at least one aromatic isocyanate comprises n chemical sub-processes sp(i). It is preferred that at least one sub-process sp(i) comprises a nitration, a hydrogenation, a phosgenation, washing, a phase separation, or a distillation.

In the case where a sub-process sp(i) comprises a nitration, this can be indicated by an index N. Thus, a sub-process comprising a nitration can be designated as sp_{N}(i). Similarly, a sub-process comprising a hydrogenation can be designated as sp_{H}(i), a sub-process comprising a phosgenation as sp_{P}(i), a sub-process comprising a washing as sp_{w}(i), a sub-process comprising a separation as sp_{S}(i), and a sub-process comprising a distillation as sp_{D}(i). The same applies to a sub-unit U(i), an educt composition E(i), a storing device D(m), a product composition P(i) or a chemical start-up composition relating to a sub-process as defined above.

It is preferred that at least one sub-process sp(i) comprises a nitration and at least one further sub-process sp(i) comprises a hydrogenation. Also, it is preferred that at least one further sub-process sp(i) comprises a phosgenation. Thus, it is particularly preferred that at least one sub-process sp(i) comprises a nitration, that at least one further sub-process sp(i) comprises a hydrogenation, and that at least one further sub-process sp(i) comprises a phosgenation.

It is preferred that the at least one aromatic isocyanate comprises an aromatic diisocyanate, preferably a toluene diisocyanate, more preferably one or more of 2,4-toluene diisocyanate and 2,6-toluene diisocyanate, or a methylene diphenyl diisocyanate, preferably one or more of methylene diphenyl 2,2'-diisocyanate, methylene diphenyl 2,4'-diisocyanate, and methylene diphenyl 4,4'-diisocyanate.

It is particularly preferred that at least one sub-process sp(i) comprises a nitration, a hydrogenation, or a phosgenation, more preferably at least one sub-process sp(i) comprises a nitration, at least one further sub-process sp(i) comprises a hydrogenation, and at least one further sub-process sp(i) comprises a phosgenation, wherein the at least one aromatic isocyanate comprises an aromatic diisocyanate, preferably a toluene diisocyanate, more preferably one or more of 2,4-toluene diisocyanate and 2,6-toluene diisocyanate, or a methylene diphenyl diisocyanate, preferably one or more of methylene diphenyl 2,2'-diisocyanate, methylene diphenyl 2,4'-diisocyanate, and methylene diphenyl 4,4'-diisocyanate. Further, it is particularly preferred that the at least one aromatic isocyanate comprises, preferably is, one or more of 2,4-toluene diisocyanate and 2,6-toluene diisocyanate.

In accordance with the above, it is preferred that one or more sub-processes sp(i) comprise a nitration, sp_{N}(i). In accordance with the above, a sub-unit U(i) in which a sub-process sp_{N}(i) is carried out is also designated herein as U_{N}(i), an educt composition relating to a sub-process sp_{N}(i) is designated herein as E_{N}(i), and a product composition P(i) obtained from a sub-unit U_{N}(i) is designated herein as P_{N}(i). Thus, it is preferred that one or more sub-processes sp(i) comprise a nitration, sp_{N}(i), wherein each sub-unit U_{N}(i) in which a sub-process sp_{N}(i) is carried out comprises a nitration reactor, wherein an educt composition E_{N}(i), being fed into a sub-unit U_{N}(i), comprises at least one nitrating agent, preferably nitric acid, and preferably further comprises at least one water scavenger, preferably sulfuric acid.

More preferably, the process according to the present invention comprises two or more sub-processes sp_{N}(i) which are carried out in sequence, wherein at last a portion of the product composition P_{N}(i) obtained from a sub-unit U_{N}(i) is fed, optionally after an intermediate treatment sub-process, as educt composition E_{N}(i+1) into the sub-unit U_{N}(i+1) downstream of U_{N}(i) during a regular operating mode of U_{N}(i+1), wherein the educt composition E_{N}(1) being fed into the first, most upstream sub-unit U_{N}(1) comprises toluene as Z¹_{E,N}(1), wherein more preferably, j=1.

Further, it is preferred that during a regular operating mode of a sub-unit U_{N}(i), a portion of P_{N}(i) is stored in at least one storing device D_{N}(m). As defined hereinabove, r storing devices D(m), m=1...r, r≥1, can be comprised in the chemical unit U in addition to the n chemical processing sub-units U(i). Thus, it is particularly preferred that that the process according to the present invention further comprises, during a regular operating mode of a sub-unit U_{N}(i), removing the product composition P_{N}(i) from said sub-unit U_{N}(i) and transferring a portion of said product composition P_{N}(i) removed from said sub-unit U_{N}(i) into a storing device D_{N}(m).

In the case where the process according to the present invention further comprises, during a regular operating mode of a sub-unit U_{N}(i), removing the product composition P_{N}(i) from said sub-unit U_{N}(i) and transferring a portion of said product composition P_{N}(i) removed from said sub-unit U_{N}(i) into a storing device D_{N}(m), it is preferred that said process comprises starting up a sub-process sp_{N}(i) in a sub-unit U_{N}(i), wherein said starting up comprises feeding at least a portion of P_{N}(i), obtained from said U_{N}(i) during regular operation mode of U_{N}(i), and stored in the at least one storing device D_{N}(m), as S_{N}(i) into U_{N}(i).

In the case where one or more sub-processes sp(i) comprise a nitration, sp_{N}(i), wherein each sub-unit U_{N}(i) in which a sub-process sp_{N}(i) is carried out comprises a nitration reactor, wherein an educt composition E_{N}(i), being fed into a sub-unit U_{N}(i), comprises at least one nitrating agent, it is preferred that a product composition P_{N}(i), obtained from a sub-unit U_{N}(i), comprises one or more of 2,4-dinitrotoluene as Z¹_{P,N}(i), 2,6-dinitrotoluene as Z²_{P,N}(i), and 2-nitrotoluene as Z³_{P,N}(i ).

Further in the case where one or more sub-processes sp(i) comprise a nitration, sp_{N}(i), wherein each sub-unit U_{N}(i) in which a sub-process sp_{N}(i) is carried out comprises a nitration reactor, wherein an educt composition E_{N}(i), being fed into a sub-unit U_{N}(i), comprises at least one nitrating agent, it is preferred that the process further comprises at least one first intermediate treatment sub-process, wherein a first intermediate treatment sub-process comprises, preferably consists of, a phase separation sub-process sp_{S}(i). Said phase separation sub-process sp_{S}(i) is carried out in a sub-unit which is also designated herein as U_{S}(i), wherein the product composi-tion P_{N}(i) obtained from U_{N}(i), prior to being fed to the sub-unit U_{N}(i+1), is subjected as educt composition E_{S}(i) to separation and is separated during sp_{S}(i) in an organic phase and an aqueous phase, wherein the organic phase, which is also designated herein as P_{S}(i), is fed as educt composition E_{N}(i+1) into the sub-unit U_{N}(i+1), wherein the sub-unit U_{S}(i) in which sp_{S}(i) is carried out comprises a phase separator.

If the process according to the present invention comprises at least one first intermediate treatment sub-process as defined above, it is preferred that a portion of P_{S}(i) is stored in at least one storing device D_{S}(m) during a regular operating mode of a sub-unit U_{S}(i). As defined hereinabove, r storing devices D(m), m=1...r, r≥1, can be comprised in the chemical unit U in addition to the n chemical processing sub-units U(i). Thus, it is particularly preferred that the process according to the present invention further comprises, during a regular operating mode of a sub-unit U_{S}(i), removing the product composition P_{S}(i) from said sub-unit U_{S}(i) and transferring a portion of said product composition P_{S}(i) removed from said sub-unit U_{S}(i) into a storing device D_{S}(m).

In the case where the process of the present invention comprises storing a portion of P_{S}(i) in at least one storing device D_{S}(m) during a regular operating mode of a sub-unit U_{S}(i), it is preferred that the process comprises starting up the sub-process sp_{S}(i) in the sub-unit U_{S}(i), wherein said starting up comprises feeding at least a portion of P_{S}(i), obtained from said U_{S}(i) during a regular operating mode of U_{S}(i) and stored in the at least one storing device D_{S}(m), as chemical start-up composition S_{S}(i) into U_{S}(i).

Further in the case where the process of the present invention comprises storing a portion of P_{S}(i) in at least one storing device D_{S}(m) during a regular operating mode of a sub-unit U_{S}(i), it is preferred that the process comprises starting up the sub-process sp_{N}(i) in a sub-unit U_{N}(i), wherein said starting up comprises feeding at least a portion of P_{S}(i), obtained from said U_{S}(i) and stored in the at least one storing device D_{N}(m), as chemical start-up composition S_{N}(i) into U_{N}(i).

In the case where the process further comprises at least one first intermediate treatment sub-process, wherein a first intermediate treatment sub-process comprises, preferably consists of, a phase separation sub-process sp_{S}(i) as defined above, it is preferred that a product composition P_{S}(i), obtained from a sub-unit U_{S}(i), comprises one or more of 2,4-dinitrotoluene as Z¹_{P,S}(i), 2,6-dinitrotoluene as Z²_{P,S}(i), and 2-nitrotoluene as Z³_{P,S}(i).

Further in the case where the process further comprises at least one first intermediate treatment sub-process, wherein a first intermediate treatment sub-process comprises, preferably consists of, a phase separation sub-process sp_{S}(i) as defined above, it is preferred that the product composition P_{S}(i), obtained from the most downstream sub-unit U_{S}(i), comprises one or more of 2,4-dinitrotoluene as Z¹_{P,S}(i) and 2,6-dinitrotoluene as Z²_{P,S}(i), wherein at least 75 mol-%, preferably at least 80 mol-%, more preferably at least 85 mol-%, more preferably at least 90 mol-% of all Z^{k}_{P,S}(i) comprised in the product composition P_{S}(i) obtained from the most downstream sub-unit U_{S}(i) consist of one or more of 2,4-dinitrotoluene as Z¹_{P,S}(i) and 2,6-dinitrotoluene as Z²_{P,S}(i).

As noted above, it is preferred that one or more sub-processes sp(i) comprise a nitration, sp_{N}(i), wherein each sub-unit U_{N}(i) in which a sub-process sp_{N}(i) is carried out comprises a nitration reactor. In this connection, it is further preferred that an educt composition E_{N}(i), being fed into a sub-unit U_{N}(i), comprises at least one nitrating agent and preferably further comprises at least one water scavenger. In such a case, it is preferred that the process further comprises at least one second intermediate treatment sub-process, wherein a second intermediate treatment sub-process comprises, preferably consists of a washing sub-process, sp_{w}(i), which is carried out in a sub-unit U_{W}(i), wherein a product composition P_{N}(i) obtained from a sub-unit U_{N}(i), or a product composition P_{S}(i) obtained from a sub-unit U_{S}(i), is subjected as educt composition E_{W}(i) to washing, obtaining a product composition P_{W}(i), wherein the sub-unit U_{W}(i) in which sp_{W}(i) is carried out comprises a washing device.

In the case where the process further comprises at least one second intermediate treatment sub-process as defined above, it is preferred that the process comprises one sub-process sp_{W}(1), wherein sp_{W}(1) is carried out directly downstream of the most downstream sub-process sp_{N}(i), or directly downstream of the most downstream sub-process sp_{S}(i), or directly downstream of the most downstream sub-process sp_{N}(i) and directly downstream of the most downstream sub-process sp_{S}(i), preferably directly downstream of the most downstream sub-process sp_{S}(i).

Further in the case where the process further comprises at least one second intermediate treatment sub-process as defined above, it is preferred that the process comprises, during a regular operating mode of a sub-unit U_{W}(i), storing a portion of P_{W}(i) in at least one storing device D_{W}(m). As defined hereinabove, r storing devices D(m), m=1...r, r≥1, can be comprised in the chemical unit U in addition to the n chemical processing sub-units U(i).

If a portion of P_{W}(i) is stored in at least one storing device D_{W}(m) during a regular operating mode of a sub-unit U_{W}(i), it is preferred that the process comprises starting up a sub-process sp_{W}(i) in a sub-unit U_{W}(i), wherein said starting up comprises feeding at least a portion of P_{W}(i), obtained from said U_{W}(i) and stored in the at least one storing device D_{W}(m), as S_{W}(i) into U_{W}(i).

Further in the case where the process further comprises at least one second intermediate treatment sub-process as defined above, it is preferred that a product composition P_{W}(i), obtained from a sub-unit U_{W}(i), comprises one or more of 2,4-dinitrotoluene as Z¹_{P,W}(i), 2,6-dinitrotoluene as Z²_{P,W}(i), and 2-nitrotoluene as Z³_{P,W}(i).

Further in the case where the process further comprises at least one second intermediate treatment sub-process as defined above, it is preferred that the product composition P_{W}(i), obtained from the most downstream sub-unit U_{W}(i), comprises one or more of 2,4-dinitrotoluene as Z¹_{P,W}(i) and 2,6-dinitrotoluene as Z²_{P,W}(i), wherein at least 75 mol-%, preferably at least 80 mol-%, more preferably at least 85 mol-%, more preferably at least 90 mol-% of all Z^{k}_{P,W}(i) comprised in the product composition P_{W}(i) obtained from the most downstream sub-unit U_{W}(i) consist of one or more of 2,4-dinitrotoluene as Z¹_{P,W}(i) and 2,6-dinitrotoluene as Z²_{P,w}(i).

It is preferred that one or more sub-processes sp(i) comprise a hydrogenation, sp_{H}(i), wherein each sub-unit U_{H}(i) in which a sub-process sp_{H}(i) is carried out comprises a hydrogenation reactor, wherein an educt composition E_{H}(i), being fed into a sub-unit U_{H}(i), comprises at least one hydrogenation agent.

It is further preferred that one or more sub-processes sp(i) comprise a nitration, sp_{N}(i), as defined herein, and that one or more sub-processes sp(i) comprise a hydrogenation, sp_{H}(i), wherein each sub-unit U_{H}(i) in which a sub-process sp_{H}(i) is carried out comprises a hydrogenation reactor, wherein an educt composition E_{H}(i), being fed into a sub-unit U_{H}(i), comprises at least one hydrogenation agent.

It is particularly preferred that one or more sub-processes sp(i) comprise a nitration, sp_{N}(i), as defined herein, that the process comprises at least one first intermediate treatment sub-process as defined herein, and that one or more sub-processes sp(i) comprise a hydrogenation, sp_{H}(i), wherein each sub-unit U_{H}(i) in which a sub-process sp_{H}(i) is carried out comprises a hydrogenation reactor, wherein an educt composition E_{H}(i), being fed into a sub-unit U_{H}(i), comprises at least one hydrogenation agent.

It is more particularly preferred that one or more sub-processes sp(i) comprise a nitration, sp_{N}(i), as defined herein, that the process comprises at least one first intermediate treatment sub-process as defined herein, that the process comprises at least one second intermediate treatment sub-process as defined herein, and that one or more sub-processes sp(i) comprise a hydrogenation, sp_{H}(i), wherein each sub-unit U_{H}(i) in which a sub-process sp_{H}(i) is carried out comprises a hydrogenation reactor, wherein an educt composition E_{H}(i), being fed into a sub-unit U_{H}(i), comprises at least one hydrogenation agent.

In the case where one or more sub-processes sp(i) comprise a hydrogenation, sp_{H}(i), as defined herein, it is preferred that the process comprises one sub-process s_{PH}(1).

Further in the case where one or more sub-processes sp(i) comprise a hydrogenation, sp_{H}(i), as defined herein, it is preferred that the at least one aromatic compound Z^{j}_{E.H}(i) comprised in E_{H}(i) is one or more of 2,4-dinitrotoluene as Z¹_{E,H}(i), 2,6-dinitrotoluene as Z²_{E,H}(i), 2-nitrotoluene as Z³_{E,H}(i) and 6-nitrotoluene as Z⁴_{E,H}(i).

Further in the case where one or more sub-processes sp(i) comprise a hydrogenation, sp_{H}(i), as defined herein, it is preferred that E_{H}(i) comprises one or more of a product composition P_{N}(i) as defined in any one of the embodiments disclosed herein, a product composition P_{S}(i) as defined in any one of the embodiments disclosed herein, and a product composition P_{W}(i) as defined in any one of the embodiments disclosed herein, preferably a product composition P_{W}(i) obtained from washing as defined in any one of the embodiments disclosed herein, preferably a product composition P_{W}(i) obtained from the most downstream sub-unit U_{W}(i), and comprising one or more of 2,4-dinitrotoluene as Z¹_{P,W}(i) and 2,6-dinitrotoluene as Z²_{P,W}(i), wherein at least 75 mol-%, preferably at least 80 mol-%, more preferably at least 85 mol-%, more preferably at least 90 mol-% of all Z^{k}_{P,W}(i) comprised in the product composition P_{W}(i) obtained from the most downstream sub-unit U_{W}(i) consist of one or more of 2,4-dinitrotoluene as Z¹_{P,W}(i) and 2,6-dinitrotoluene as Z²_{P,w}(i).

Further in the case where one or more sub-processes sp(i) comprise a hydrogenation, sp_{H}(i), as defined herein, it is preferred that the process comprises a portion of P_{H}(i) is stored in at least one storing device D_{H}(m) during a regular operating mode of a sub-unit U_{H}(i). As defined hereinabove, r storing devices D(m), m=1...r, r≥1, can be comprised in the chemical unit U in addition to the n chemical processing sub-units U(i).

If a portion of P_{H}(i) is stored in at least one storing device D_{H}(m) during a regular operating mode of a sub-unit U_{H}(i), it is preferred that the process comprises starting up a sub-process sp_{H}(i) in a sub-unit U_{H}(i), wherein said starting up comprises feeding at least a portion of P_{H}(i), obtained from said U_{H}(i) and stored in the at least one storing device D_{H}(M), as S_{H}(i) into U_{H}(i).

Further in the case where one or more sub-processes sp(i) comprise a hydrogenation, sp_{H}(i), as defined herein, it is preferred that a product composition P_{H}(i), obtained from a sub-unit U_{H}(i), comprises one or more of 2,4-diaminotoluene as Z¹_{P,H}(i), 2,6-diaminotoluene as Z²_{P,H}(i), and 2-aminotoluene as Z³_{P,H}(i).

Further in the case where one or more sub-processes sp(i) comprise a hydrogenation, sp_{H}(i), as defined herein, it is preferred that the process comprises starting up a sub-process sp_{H}(i) in a sub-unit U_{H}(i), said starting up comprising feeding at least a portion of P_{D}(i), obtained from U_{D}(i) and stored in at least one storing device D_{D,P}(m) as defined hereinbelow, as S_{H}(i) into U_{H}(i).

Further in the case where one or more sub-processes sp(i) comprise a hydrogenation, sp_{H}(i), as defined herein, it is preferred that the process comprises starting up a sub-process sp_{H}(i) in a sub-unit U_{H}(i), wherein said starting up comprises feeding at least a portion of U_{D}(i), obtained from U_{D}(i) and stored in at least one storing device D_{D,C}(m) as defined hereinbelow, as S_{H}(i) into U_{H}(i).

Further in the case where one or more sub-processes sp(i) comprise a hydrogenation, sp_{H}(i), as defined herein, it is preferred that the process further comprises at least one third intermediate treatment sub-process, wherein a third intermediate treatment sub-process comprises, preferably consists of, a distillation sub-process, s_{PD}(i), which is carried out in a sub-unit U_{D}(i), wherein preferably, a product composition P_{H}(i) obtained from a sub-unit U_{H}(i) is subjected as educt composition E_{D}(i) to distillation, obtaining a product composition P_{D}(i), wherein the sub-unit U_{D}(i) in which sp_{D}(i) is carried out comprises a distillation device and wherein, in U_{D}(i), E_{D}(i) is separated in P_{D}(i) and at least one further distillation composition C_{D}(i).

If the process further comprises at least one third intermediate treatment sub-process as defined above, it is preferred that a portion of P_{D}(i) is stored in at least one storing device D_{D,P}(m) during a regular operating mode of a sub-unit U_{D}(i). As defined hereinabove, r storing devices D(m), m=1...r, r≥1, can be comprised in the chemical unit U in addition to the n chemical processing sub-units U(i).

In the case where a portion of P_{D}(i) is stored in at least one storing device D_{D,P}(m) during a regular operating mode of a sub-unit U_{D}(i), it is preferred that the process comprises starting up a sub-process sp_{D}(i) in a sub-unit U_{D}(i), wherein said starting up comprises feeding at least a portion of P_{D}(i), obtained from said U_{D}(i) and stored in the at least one storing device D_{D,P}(m), as S_{D}(i) into U_{D}(i).

If the process comprises starting up a sub-process sp_{H}(i) in a sub-unit U_{H}(i), wherein said starting up comprises feeding at least a portion of P_{D}(i), obtained from U_{D}(i) and stored in at least one storing device D_{D,P}(m) as defined hereinabove, as S_{H}(i) into U_{H}(i), it is preferred that a portion of C_{D}(i) is stored in at least one storing device D_{D,C}(m) during a regular operating mode of a sub-unit U_{D}(i). As defined hereinabove, r storing devices D(m), m=1...r, r≥1, can be comprised in the chemical unit U in addition to the n chemical processing sub-units U(i).

In the case where a portion of C_{D}(i) is stored in at least one storing device D_{D,C}(m) during a regular operating mode of a sub-unit U_{D}(i), it is preferred that the process comprises starting up a sub-process sp_{D}(i) in a sub-unit U_{D}(i), wherein said starting up comprises feeding at least a portion of C_{D}(i), obtained from said U_{D}(i) and stored in the at least one storing device D_{D,C}(m), as S_{D}(i) into U_{D}(i).

If the process comprises starting up a sub-process sp_{H}(i) in a sub-unit U_{H}(i), wherein said starting up comprises feeding at least a portion of P_{D}(i), obtained from U_{D}(i) and stored in at least one storing device D_{D,P}(m) as defined hereinabove, as S_{H}(i) into U_{H}(i), it is preferred that a product composition P_{D}(i), obtained from a sub-unit U_{D}(i), comprises one or more of 2,4-diaminotoluene as Z¹_{P,D}(i), 2,6-diaminotoluene as Z²_{P,D}(i), and 2-aminotoluene as Z³_{P,D}(i).

It is preferred that the weight ratio of the total weight of 2,4-diaminotoluene as Z¹_{P,D}(i), 2,6-diaminotoluene as Z²_{P,D}(i), and 2-aminotoluene as Z³_{P,D}(i) comprised in P_{D}(i) relative to the total weight of 2,4-diaminotoluene as Z¹_{P,H}(i), 2,6-diaminotoluene as Z²_{P,H}(i), and 2-aminotoluene as Z³_{P,H}(i) comprised in P_{H}(i) is greater than 1:1, more preferably in the range of from 1.05:1 to 2.5:1, more preferably in the range of from 1.3:1 to 2.2:1, more preferably in the range of from 1.5:1 to 2.1:1.

It is preferred that the weight ratio of the product composition P_{D}(i) relative to the at least one further distillation composition C_{D}(i) is in the range of from 25:1 to 2:1, more preferably in the range of from 20:1 to 5:1.

Further in the case where the process comprises starting up a sub-process sp_{H}(i) in a sub-unit U_{H}(i), wherein said starting up comprises feeding at least a portion of P_{D}(i), obtained from U_{D}(i) and stored in at least one storing device D_{D,P}(m) as defined hereinabove, as S_{H}(i) into U_{H}(i), it is preferred that the process comprises starting up a sub-process sp_{D}(i) in a sub-unit U_{D}(i), wherein said starting up comprises feeding at least a portion of P_{H}(i), obtained from U_{H}(i) and stored in the at least one storing device D_{H}(m) as defined herein, as S_{D}(i) into U_{D}(i).

As defined above, it is preferred that one or more sub-processes sp(i) comprise a nitration, sp_{N}(i), wherein each sub-unit U_{N}(i) in which a sub-process sp_{N}(i) is carried out comprises a nitration reactor, wherein an educt composition E_{N}(i), being fed into a sub-unit U_{N}(i), comprises at least one nitrating agent, preferably nitric acid, and preferably further comprises at least one water scavenger, preferably sulfuric acid. In such a case, it is preferred that one or more sub-processes sp(i) comprise a phosgenation, sp_{P}(i), wherein each sub-unit U_{P}(i) in which a sub-process sp_{P}(i) is carried out comprises a phosgenation reactor, wherein an educt composition E_{P}(i), being fed into a sub-unit U_{P}(i), comprises at least one phosgenation agent.

As defined herein, it is preferred that one or more sub-processes sp(i) comprise a nitration, sp_{N}(i). In such a case, it is preferred that one or more sub-processes sp(i) comprise a phosgenation, sp_{P}(i), wherein each sub-unit U_{P}(i) in which a sub-process sp_{P}(i) is carried out comprises a phosgenation reactor, wherein an educt composition E_{P}(i), being fed into a sub-unit U_{P}(i), comprises at least one phosgenation agent.

It is further preferred that one or more sub-processes sp(i) comprise a nitration, sp_{N}(i), as defined herein, that the process comprises at least one first intermediate treatment sub-process as defined herein, and that one or more sub-processes sp(i) comprise a phosgenation, sp_{P}(i), wherein each sub-unit U_{P}(i) in which a sub-process sp_{P}(i) is carried out comprises a phosgenation reactor, wherein an educt composition E_{P}(i), being fed into a sub-unit U_{P}(i), comprises at least one phosgenation agent.

It is further preferred that one or more sub-processes sp(i) comprise a nitration, sp_{N}(i), as defined herein, that the process comprises at least one first intermediate treatment sub-process as defined herein, that the process comprises at least one second intermediate treatment sub-process as defined herein, and that one or more sub-processes sp(i) comprise a phosgenation, sp_{P}(i), wherein each sub-unit U_{P}(i) in which a sub-process sp_{P}(i) is carried out comprises a phosgenation reactor, wherein an educt composition E_{P}(i), being fed into a sub-unit U_{P}(i), comprises at least one phosgenation agent.

It is further preferred that one or more sub-processes sp(i) comprise a nitration, sp_{N}(i), as defined herein, that the process comprises at least one first intermediate treatment sub-process as defined herein, that the process comprises at least one second intermediate treatment sub-process as defined herein, that one or more sub-processes sp(i) comprise a hydrogenation, sp_{H}(i), as defined herein, and that one or more sub-processes sp(i) comprise a phosgenation, sp_{P}(i), wherein each sub-unit U_{P}(i) in which a sub-process sp_{P}(i) is carried out comprises a phosgenation reactor, wherein an educt composition E_{P}(i), being fed into a sub-unit U_{P}(i), comprises at least one phosgenation agent.

It is further preferred that one or more sub-processes sp(i) comprise a nitration, sp_{N}(i), as defined herein, that the process comprises at least one first intermediate treatment sub-process as defined herein, that the process comprises at least one second intermediate treatment sub-process as defined herein, that one or more sub-processes sp(i) comprise a hydrogenation, sp_{H}(i), as defined herein, that the process further comprises at least one third intermediate treatment sub-process as defined herein, and that one or more sub-processes sp(i) comprise a phosgenation, sp_{P}(i), wherein each sub-unit U_{P}(i) in which a sub-process sp_{P}(i) is carried out comprises a phosgenation reactor, wherein an educt composition E_{P}(i), being fed into a sub-unit U_{P}(i), comprises at least one phosgenation agent.

If one or more sub-processes sp(i) comprise a phosgenation, sp_{P}(i), as defined herein, it is preferred that the process comprises one sub-process sp_{P}(1).

Further in the case where one or more sub-processes sp(i) comprise a phosgenation, sp_{P}(i), as defined herein, it is preferred that the at least one aromatic compound Z^{j}_{E.P}(i) comprised in E_{P}(i) is one or more of 2,4-diaminotoluene as Z¹_{E,P}(i), 2,6-diaminotoluene as Z²_{E,P}(i), 2-aminotoluene as Z³_{E,P}(i) and 6-aminotoluene as Z⁴_{E,P}(i).

Further in the case where one or more sub-processes sp(i) comprise a phosgenation, sp_{P}(i), as defined herein, it is preferred that E_{P}(i) comprises one or more of a product composition P_{H}(i) as defined in any one of the embodiments disclosed herein, a product composition P_{D}(i) as defined in any one of the embodiments disclosed herein, a product composition P_{D}(i) as defined in any one of the embodiments disclosed herein, a distillation composition C_{D}(i) as defined in any one of the embodiments disclosed herein, preferably a product composition P_{D}(i) obtained from a sub-unit U_{D}(i) as defined herein or a product composition P_{D}(i) obtained from the most downstream sub-unit U_{D}(i) as defined herein, more preferably a product composition P_{D}(i) obtained from the most downstream sub-unit U_{D}(i) as defined herein.

In the case where one or more sub-processes sp(i) comprise a phosgenation, sp_{P}(i), as defined herein, it is preferred that a portion of P_{P}(i) is stored in at least one storing device D_{P}(m) during a regular operating mode of a sub-unit U_{P}(i). As defined hereinabove, r storing devices D(m), m=1...r, r≥1, can be comprised in the chemical unit U in addition to the n chemical processing sub-units U(i).

Further in the case where one or more sub-processes sp(i) comprise a phosgenation, sp_{P}(i), as defined herein, it is preferred that a portion of P_{P}(i) is stored in at least one storing device Dₑ(m) during a regular operating mode of a sub-unit U_{P}(i). As defined hereinabove, r storing devices D(m), m=1...r, r≥1, can be comprised in the chemical unit U in addition to the n chemical processing sub-units U(i).

If a portion of P_{P}(i) is stored in at least one storing device D_{P}(m) during a regular operating mode of a sub-unit U_{P}(i), it is preferred that the process comprises starting up a sub-process sp_{P}(i) in a sub-unit U_{P}(i), wherein said starting up comprises feeding at least a portion of P_{P}(i), obtained from said U_{D}(i)and stored in the at least one storing device D_{P}(m), as S_{P}(i) into U_{P}(i).

Further in the case where one or more sub-processes sp(i) comprise a phosgenation, sp_{P}(i), as defined herein, it is preferred that a product composition P_{P}(i), obtained from a sub-unit U_{P}(i), comprises one or more of 2,4-toluene diisocyanate as Z¹_{P,P}(i), 2,6-toluene diisocyanate as Z²_{P,P}(i), and 2-toluene isocyanate as Z³_{P,P}(i).

It is preferred that the abovementioned process is a continuous process or discontinuous process, more preferably a continuous process.

Further, the present invention relates to a chemical unit U for carrying out the process according to any one of the embodiments defined herein, wherein
the chemical unit comprises
(i.1) one or more sub-units U_{N}(i), preferably two sub-units U_{N}(1) and U_{N}(2);
(i.2) one or more sub-units U_{S}(i), preferably two sub-units U_{S}(1) and U_{S}(2);
(i.3) one or more sub-units U_{W}(i), preferably one sub-unit U_{W}(1);
(i.4) one or more storing devices D_{N}(m), preferably two storing devices D_{N}(1) and D_{N}(2);
   wherein U_{N}(1) is arranged upstream of U_{S}(1), U_{S}(1) is arranged upstream U_{N}(2), U_{N}(2) is arranged upstream U_{S}(2), and U_{S}(2) is arranged upstream of U_{W}(1);
(ii.1) one or more sub-units U_{H}(i), preferably one sub-unit U_{H}(1);
(ii.2) one or more sub-units U_{D}(i), preferably one sub-unit U_{D}(1);
(ii.3) one or more storing devices D_{H}(m), preferably one storing device D_{H}(1);
(ii.4) one or more storing devices D_{D}(m), preferably two storing devices D_{D,P}(1) and D_{D,C}(1);
   wherein U_{H}(1) is arranged downstream of U_{W}(1) and upstream U_{D}(1);
(iii.1) one or more sub-units U_{P}(i), preferably one sub-unit U_{P}(1);
   wherein U_{P}(1) is arranged downstream of U_{D}(1).

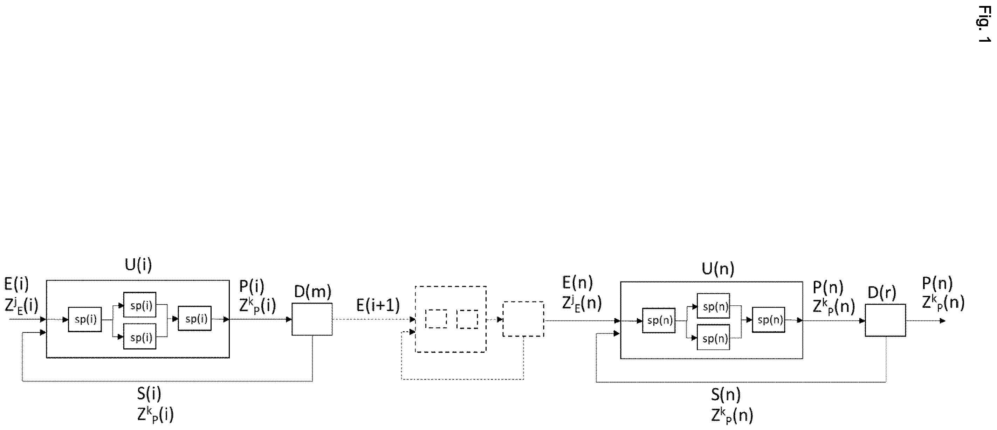

Figure 1 shows a flow diagram according to the process of the present invention. Depicted is a process comprising n chemical sub-processes sp(i). The sub-process sp(i) is carried out in a chemical processing sub-unit U(i) where i=1...n, with n≥1.

For example, when n=1, during regular operation mode of a sub-unit U_{N}(1) for nitration, the sub-process sp_{N}(1) being carried out in said sub-unit U_{N}(1) comprises feeding an educt composition E_{N}(1) comprising at least one aromatic compound Z^{j}_{E}(1), such as toluene, into said sub-unit U_{N}(1). The composition E_{N}(1) comprising at least one aromatic compound Z^{j}_{E}(1) is processed in said sub-unit to obtain a product composition P_{N}(1) comprising at least one aromatic compound Z^{k}_{P}(1), k≥1 being dinitrotoluene.

For example, when n=2, for nitration, two sub-units U_{N}(1) and U_{N}(2), the sub-units U_{N}(1) and U_{N}(2), are part of a chemical unit U, wherein U further comprises, in addition to the 2 chemical processing sub-units U_{N}(1) and U_{N}(2), one storing device D_{N}(1). During regular operation mode of the sub-unit U_{N}(1), a portion of the product P_{N}(1) obtained from U_{N}(1) and comprising dinitrotoluene is stored in a storing device D_{N}(1), and the remaining portion of the product P_{N}(1) is removed from U_{N}(1) and fed to either U_{N}(2) or to a further downstream sub-unit. Sub-unit U_{N}(1) is shut down for maintenance and regular operation is stopped. After a maintenance routine of U_{N}(1) is completed, in the starting up process of the sub-process sp_{N}(1) in sub-unit U_{N}(1), a portion of P_{N}(1) (dinitrotoluene), obtained from said U_{N}(1) during regular operation mode of U_{N}(1) and U_{N}(2), and stored in the storing device D_{N}(1), is fed back into the nitration sub-unit U_{N}(1) as a start-up composition S_{N}(1). Accordingly, the start-up composition S_{N}(1) ≠ educt composition E_{N}(1). Depicted as well are optional intermediate processes and units symbolized as a broken lines between the two sub-units.

### Cited literature

- WO 2015/197522 A1
- WO 2017/050776 A1

## Claims

1. A chemical process for preparing at least one aromatic isocyanate, said process comprising n chemical sub-processes sp(i), wherein a sub-process sp(i) is carried out in a chemical processing sub-unit U(i), i=1...n, n≥1,
wherein during a regular operating mode of a sub-unit U(i), the sub-process sp(i) being carried out in said sub-unit U(i) comprises feeding an educt composition E(i) comprising at least one aromatic compound Z^{j}_{E}(i), j≥1, into said sub-unit U(i), processing said composition E(i) in said sub-unit U(i) and obtaining a product composition P(i) comprising at least one aromatic compound Z^{k}_{P}(i), k≥1,
wherein the chemical process comprises starting up said sub-process sp(i) in said sub-unit U(i), wherein said starting-up comprises processing a chemical start-up composition S(i) in said sub-unit U(i), wherein said S(i) ≠ E(i) and wherein said S(i) comprises at least one of the aromatic compounds Z^{k}ₚ(j).

2. The process of claim 1, wherein, if n≥2, at least 2, preferably all sub-units U(i) are part of a chemical unit U, wherein U further comprises, in addition to the n chemical processing sub-units U(i), r storing devices D(m), m=1...r, r≥1;
said chemical process further comprising, during a regular operating mode of a sub-unit U(i), removing the product composition P(i) from said sub-unit U(i) and transferring a portion of said product composition P(i) removed from said sub-unit U(i) into a storing device D(m);
said chemical process further comprising, during starting up the sub-process sp(i), feeding at least a portion of P(i), stored in D(m) during the regular operating mode of the sub-unit U(i), as at least a portion of the chemical composition S(i) into the sub-unit U(i).

3. The process of claim 1 or 2, wherein the sub-unit U(i) is selected from the group consisting of a chemical reactor, a distillation column, a rectification column, a tank, a phase separator, a washing device, and a combination of two or more thereof, wherein at least one sub-process sp(i) comprises a nitration and at least one further sub-process sp(i) comprises a hydrogenation; and wherein the at least one aromatic isocyanate preferably comprises an aromatic diisocyanate, more preferably a toluene diisocyanate, more preferably one or more of 2,4-toluene diisocyanate and 2,6-toluene diisocyanate, or a methylene diphenyl diisocyanate, more preferably one or more of methylene diphenyl 2,2'-diisocyanate, methylene diphenyl 2,4'-diisocyanate, and methylene diphenyl 4,4'-diisocyanate; wherein more preferably, the at least one aromatic isocyanate comprises, preferably is, one or more of 2,4-toluene diisocyanate and 2,6-toluene diisocyanate.

4. The process of any one of claims 1 to 3, preferably of claim 3, wherein one or more sub-processes sp(i) comprise a nitration, sp_{N}(i), wherein each sub-unit U_{N}(i) in which a sub-process sp_{N}(i) is carried out comprises a nitration reactor, wherein an educt composition E_{N}(i), being fed into a sub-unit U_{N}(i), comprises at least one nitrating agent, preferably nitric acid, and preferably further comprises at least one water scavenger, preferably sulfuric acid;
wherein said process preferably comprises two or more sub-processes sp_{N}(i) which are carried out in sequence, wherein at last a portion of the product composition P_{N}(i) obtained from a sub-unit U_{N}(i) is fed, optionally after an intermediate treatment sub-process, as educt composition E_{N}(i+1) into the sub-unit U_{N}(i+1) downstream of U_{N}(i) during a regular operating mode of U_{N}(i+1);
wherein the educt composition E_{N}(1) being fed into the first, most upstream sub-unit U_{N}(1) comprises toluene as Z¹_{E,N}(1), wherein more preferably, j=1;
wherein said chemical process comprises, during a regular operating mode of a sub-unit U_{N}(i), storing a portion of P_{N}(i) in at least one storing device D_{N}(m) as defined in claim 2;
wherein said chemical process comprises, starting up a sub-process sp_{N}(i) in a sub-unit U_{N}(i), said starting up comprising feeding at least a portion of P_{N}(i), obtained from said U_{N}(i) during regular operation mode of U_{N}(i), and stored in the at least one storing device D_{N}(m), as S_{N}(i) into U_{N}(i), wherein preferably, a product composition P_{N}(i), obtained from a sub-unit U_{N}(i), comprises one or more of 2,4-dinitrotoluene as Z¹_{P,N}(i), 2,6-dinitrotoluene as Z²_{P,N}(i), and 2-nitrotoluene as Z³_{P,N}(i).

5. The process of claim 4, further comprising at least one first intermediate treatment sub-process, wherein a first intermediate treatment sub-process comprises, preferably consists of a phase separation sub-process, sp_{S}(i), which is carried out in a sub-unit U_{S}(i), wherein the product composition P_{N}(i) obtained from U_{N}(i), prior to being fed to the sub-unit U_{N}(i+1), is subjected as educt composition E_{S}(i) to separation and is separated during sp_{S}(i) in an organic phase and an aqueous phase, wherein the organic phase, P_{S}(i), is fed as educt composition E_{N}(i+1) into the sub-unit U_{N}(i+1), wherein the sub-unit U_{S}(i) in which sp_{S}(i) is carried out comprises a phase separator;
wherein said chemical comprises, during a regular operating mode of a sub-unit U_{S}(i), storing a portion of P_{S}(i) in at least one storing device D_{S}(m) as defined in claim 2;
wherein said chemical comprises starting up the sub-process sp_{S}(i) in the sub-unit U_{S}(i), said starting up comprising feeding at least a portion of P_{S}(i), obtained from said U_{S}(i) during a regular operating mode of U_{S}(i) and stored in the at least one storing device D_{S}(m), as S_{S}(i) into U_{S}(i); preferably comprising starting up the sub-process sp_{N}(i) in a sub-unit U_{N}(i), said starting up comprising feeding at least a portion of P_{S}(i), obtained from said U_{S}(i) and stored in the at least one storing device D_{N}(m), as S_{N}(i) into U_{N}(i).

6. The process of claim 5, wherein a product composition P_{S}(i), obtained from a sub-unit U_{S}(i), comprises one or more of 2,4-dinitrotoluene as Z¹_{P,S}(i), 2,6-dinitrotoluene as Z²_{P,S}(i), and 2-nitrotoluene as Z³_{P,S}(i);
wherein the product composition P_{S}(i), obtained from the most downstream sub-unit U_{S}(i), preferably comprises one or more of 2,4-dinitrotoluene as Z¹_{P,S}(i) and 2,6-dinitrotoluene as Z²_{P,S}(i), wherein preferably at least 75 mol-%, more preferably at least 80 mol-%, more preferably at least 85 mol-%, more preferably at least 90 mol-% of all Z^{k}_{P,S}(i) comprised in the product composition P_{S}(i) obtained from the most downstream sub-unit U_{S}(i) consist of one or more of 2,4-dinitrotoluene as Z¹_{P,S}(i) and 2,6-dinitrotoluene as Z²_{P,S}(i).

7. The process of claim 5 or 6, further comprising at least one second intermediate treatment sub-process, wherein a second intermediate treatment sub-process comprises, preferably consists of a washing sub-process, sp_{W}(i), which is carried out in a sub-unit U_{W}(i), wherein a product composition P_{N}(i) obtained from a sub-unit U_{N}(i), or a product composition P_{S}(i) obtained from a sub-unit U_{S}(i), is subjected as educt composition E_{W}(i) to washing, obtaining a product composition P_{W}(i), wherein the sub-unit U_{W}(i) in which sp_{W}(i) is carried out comprises a washing device;
wherein said process preferably comprises one sub-process sp_{W}(1), wherein sp_{W}(1) is carried out directly downstream of the most downstream sub-process sp_{N}(i), or directly downstream of the most downstream sub-process sp_{S}(i), or directly downstream of the most downstream sub-process sp_{N}(i) and directly downstream of the most downstream sub-process sp_{S}(i), preferably directly downstream of the most downstream sub-process sp_{S}(i);
wherein said process comprises, during a regular operating mode of a sub-unit U_{W}(i), storing a portion of P_{W}(i) in at least one storing device D_{W}(m) as defined in claim 2;
wherein said process comprises starting up a sub-process sp_{W}(i) in a sub-unit U_{W}(i), said starting up comprising feeding at least a portion of P_{W}(i), obtained from said U_{W}(i) and stored in the at least one storing device D_{W}(m), as S_{W}(i) into U_{W}(i).

8. The process of claim 7, wherein a product composition P_{W}(i), obtained from a sub-unit U_{W}(i), comprises one or more of 2,4-dinitrotoluene as Z¹_{P,W}(i), 2,6-dinitrotoluene as Z²_{P,W}(i), and 2-nitrotoluene as Z³_{P,W}(i);
wherein the product composition P_{W}(i), obtained from the most downstream sub-unit U_{W}(i), preferably comprises one or more of 2,4-dinitrotoluene as Z¹_{P,W}(i) and 2,6-dinitrotoluene as Z²_{P,W}(i), wherein preferably at least 75 mol-%, more preferably at least 80 mol-%, more preferably at least 85 mol-%, more preferably at least 90 mol-% of all Z^{k}_{P,W}(i) comprised in the product composition P_{W}(i) obtained from the most downstream sub-unit U_{W}(i) consist of one or more of 2,4-dinitrotoluene as Z¹_{P,W}(i) and 2,6-dinitrotoluene as Z²_{P,w}(i).

9. The process of any one of claims 1 to 8, preferably of any one of claims 4 to 8, more preferably of any one of claims 5 to 8, more preferably of claim 7 or 8, wherein one or more sub-processes sp(i) comprise a hydrogenation, sp_{H}(i), wherein each sub-unit U_{H}(i) in which a sub-process sp_{H}(i) is carried out comprises a hydrogenation reactor, wherein an educt composition E_{H}(i), being fed into a sub-unit U_{H}(i), comprises at least one hydrogenation agent;
wherein said process preferably comprises one sub-process sp_{H}(1);
wherein the at least one aromatic compound Z^{j}_{E.H}(i) comprised in E_{H}(i) is one or more of 2,4-dinitrotoluene as Z¹_{E,H}(i), 2,6-dinitrotoluene as Z²_{E,H}(i), 2-nitrotoluene as Z³_{E,H}(i) and 6-nitrotoluene as Z⁴_{E,H}(i);
wherein E_{H}(i) preferably comprises one or more of a product composition P_{N}(i) as defined in claim 4, a product composition P_{S}(i) as defined in claim 6, and a product composition P_{W}(i) as defined in claim 7, preferably a product composition P_{W}(i) as defined in claim 7; wherein said process comprises, during a regular operating mode of a sub-unit U_{H}(i), storing a portion of P_{H}(i) in at least one storing device D_{H}(m) as defined in claim 2;
wherein said process comprises starting up a sub-process sp_{H}(i) in a sub-unit U_{H}(i), said starting up comprising feeding at least a portion of P_{H}(i), obtained from said U_{H}(i) and stored in the at least one storing device D_{H}(m), as S_{H}(i) into U_{H}(i).

10. The process of claim 9, wherein a product composition P_{H}(i), obtained from a sub-unit U_{H}(i), comprises one or more of 2,4-diaminotoluene as Z¹_{P,H}(i), 2,6-diaminotoluene as Z²_{P,H}(i), and 2-aminotoluene as Z³_{P,H}(i).

11. The process of any one of claims 7 to 10, further comprising at least one third intermediate treatment sub-process, wherein a third intermediate treatment sub-process comprises, preferably consists of a distillation sub-process, sp_{P}(i), which is carried out in a sub-unit U_{D}(i), wherein preferably, a product composition P_{H}(i) obtained from a sub-unit U_{H}(i) is subjected as educt composition E_{D}(i) to distillation, obtaining a product composition P_{D}(i), wherein the sub-unit U_{D}(i) in which sp_{D}(i) is carried out comprises a distillation device and wherein in U_{D}(i), E_{D}(i) is separated in P_{D}(i) and at least one further distillation composition C_{D}(i);
wherein
- said process comprises, during a regular operating mode of a sub-unit U_{D}(i), storing a portion of P_{D}(i) in at least one storing device D_{D,P}(m) as defined in claim 2;
said process comprising starting up a sub-process sp_{D}(i) in a sub-unit U_{D}(i), said starting up comprising feeding at least a portion of P_{D}(i), obtained from said U_{D}(i) and stored in the at least one storing device D_{D,P}(m), as S_{D}(i) into U_{D}(i);
and/or
- said process comprises, during a regular operating mode of a sub-unit U_{D}(i), storing a portion of C_{D}(i) in at least one storing device D_{D,C}(m) as defined in claim 2;
said process comprising starting up a sub-process sp_{D}(i) in a sub-unit U_{D}(i), said starting up comprising feeding at least a portion of C_{D}(i), obtained from said U_{D}(i) and stored in the at least one storing device D_{D,C}(m), as S_{D}(i) into U_{P}(i).

12. The process of claim 11, comprising
- starting up a sub-process sp_{H}(i) in a sub-unit U_{H}(i), said starting up comprising feeding at least a portion of P_{D}(i), obtained from U_{D}(i) and stored in at least one storing device D_{D,P}(m) as defined in claim 11, as S_{H}(i) into U_{H}(i);
and/or
- starting up a sub-process sp_{H}(i) in a sub-unit U_{H}(i), said starting up comprising feeding at least a portion of U_{D}(i), obtained from U_{D}(i)and stored in at least one storing device D_{D,C}(m) as defined in claim 11, as S_{H}(i) into U_{H}(i).

13. The process of claim 11 or 12, wherein a product composition P_{D}(i), obtained from a sub-unit U_{D}(i), comprises one or more of 2,4-diaminotoluene as Z¹_{P,D}(i), 2,6-diaminotoluene as Z²_{P,D}(i), and 2-aminotoluene as Z³_{P,D}(i).

14. The process of any one of claims 11 to 13, comprising starting up a sub-process sp_{D}(i) in a sub-unit U_{D}(i), said starting up comprising feeding at least a portion of P_{H}(i), obtained from U_{H}(i) and stored in the at least one storing device D_{H}(m) as defined in claim 9, as S_{D}(i) into U_{D}(i).

15. A chemical unit U for carrying out the process according to any one of claims 1 to 14, wherein said chemical unit U comprises
(i.1) one or more sub-units U_{N}(i), preferably two sub-units U_{N}(1) and U_{N}(2);
(i.2) one or more sub-units U_{S}(i), preferably two sub-units U_{S}(1) and U_{S}(2);
(i.3) one or more sub-units U_{W}(i), preferably one sub-unit U_{W}(1);
(i.4) one or more storing devices D_{N}(m), preferably two storing devices D_{N}(1) and D_{N}(2);
wherein U_{N}(1) is arranged upstream of U_{S}(1), U_{S}(1) is arranged upstream U_{N}(2), U_{N}(2) is arranged upstream U_{S}(2), and U_{S}(2) is arranged upstream of U_{W}(1);
(ii.1) one or more sub-units U_{H}(i), preferably one sub-unit U_{H}(1);
(ii.2) one or more sub-units U_{D}(i), preferably one sub-unit U_{D}(1);
(ii.3) one or more storing devices D_{H}(m), preferably one storing device D_{H}(1);
(ii.4) one or more storing devices D_{D}(m), preferably two storing devices D_{D,P}(1) and D_{D,C}(1);
wherein U_{H}(1) is arranged downstream of U_{W}(1) and upstream U_{D}(1);
(iii.1) one or more sub-units U_{P}(i), preferably one sub-unit Up(1);
wherein U_{P}(1) is arranged downstream of U_{D}(1).

## Patentansprüche

1. Ein chemisches Verfahren zur Herstellung mindestens eines aromatischen Isocyanats, wobei das Verfahren n chemische Teilprozesse sp(i) umfasst, wobei ein Teilprozess sp(i) in einer chemischen Verarbeitungsteileinheit U(i), i=1... n, n≥1, durchgeführt wird,
wobei während eines regulären Betriebsmodus einer Teileinheit U(i) der in der Teileinheit U(i) durchgeführte Teilprozess sp(i) das Zuführen einer Edukt-Zusammensetzung E(i), die mindestens eine aromatische Verbindung Z^{j}_{E}(i), j≥1, umfasst, in die Teileinheit U(i), die Verarbeitung der Zusammensetzung E(i) in der Teileinheit U(i) und das Erhalten einer Produkt-Zusammensetzung P(i), die mindestens eine aromatische Verbindung Z^{k}_{P}(i), k≥1, umfasst, beinhaltet,
wobei das chemische Verfahren das Anfahren des Teilprozesses sp(i) in der Teileinheit U(i) umfasst, wobei das Anfahren die Verarbeitung einer chemischen Anfahrzusammensetzung S(i) in der Teileinheit U(i) umfasst, wobei S(i) ≠ E(i) und wobei S(i) mindestens eine der aromatischen Verbindungen Z^{k}_{P}(i) umfasst.

2. Das Verfahren nach Anspruch 1, wobei, wenn n≥2, mindestens 2, vorzugsweise alle Teileinheiten U(i) Teil einer chemischen Einheit U sind, wobei U zusätzlich zu den n chemischen Verarbeitungsteileinheiten U(i) r Speichervorrichtungen D(m), m=1... r, r≥1, umfasst;
wobei das chemische Verfahren ferner umfasst, während eines regulären Betriebsmodus einer Teileinheit
U(i), das Entfernen der Produkt-Zusammensetzung P(i) aus der Teileinheit U(i) und das Übertragen eines Teils der aus der Teileinheit U(i) entfernten Produkt-Zusammensetzung P(i) in eine Speichervorrichtung D(m);
wobei das chemische Verfahren ferner umfasst, während des Anfahrens des Teilprozesses sp(i), das Zuführen mindestens eines Teils von P(i), der während des regulären Betriebsmodus der Teileinheit U(i) in D(m) gespeichert wurde, als mindestens einen Teil der chemischen Zusammensetzung S(i) in die Teileinheit U(i).

3. Das Verfahren nach Anspruch 1 oder 2, wobei die Teileinheit U(i) aus der Gruppe ausgewählt ist, die aus einem chemischen Reaktor, einer Destillationskolonne, einer Rektifikationskolonne, einem Tank, einem Phasenabscheider, einer Waschvorrichtung und einer Kombination von zwei oder mehr davon besteht, wobei mindestens ein Teilprozess sp(i) eine Nitrierung und mindestens ein weiterer Teilprozess sp(i) eine Hydrierung umfasst; und wobei das mindestens eine aromatische Isocyanat vorzugsweise ein aromatisches Diisocyanat, bevorzugter ein Toluoldiisocyanat, noch bevorzugter eines oder mehrere von 2,4-Toluoldiisocyanat und 2,6-Toluoldiisocyanat, oder ein Methylendiphenyldiisocyanat, noch bevorzugter eines oder mehrere von Methylendiphenyl-2,2'-diisocyanat, Methylendiphenyl-2,4'-diisocyanat und Methylendiphenyl-4,4'-diisocyanat umfasst; wobei noch bevorzugter das mindestens eine aromatische Isocyanat eines oder mehrere von 2,4-Toluoldiisocyanat und 2,6-Toluoldiisocyanat umfasst, vorzugsweise ist.

4. Das Verfahren nach einem der Ansprüche 1 bis 3, vorzugsweise nach Anspruch 3, wobei ein oder mehrere Teilprozesse sp(i) eine Nitrierung, sp_{N}(i), umfassen, wobei jede Teileinheit U_{N}(i), in der ein Teilprozess sp_{N}(i) durchgeführt wird, einen Nitrierungsreaktor umfasst, wobei eine Edukt-Zusammensetzung E_{N}(i), die in eine Teileinheit U_{N}(i) eingespeist wird, mindestens ein Nitrierungsmittel, vorzugsweise
Salpetersäure, umfasst und vorzugsweise ferner mindestens ein Wasserentzugsmittel, vorzugsweise Schwefelsäure, umfasst;
wobei das Verfahren vorzugsweise zwei oder mehr Teilprozesse sp_{N}(i) umfasst, die nacheinander durchgeführt werden, wobei zumindest ein Teil der Produkt-Zusammensetzung P_{N}(i), die aus einer Teileinheit U_{N}(i) erhalten wird, gegebenenfalls nach einem zwischengeschalteten Behandlungsteilprozess, als Edukt-Zusammensetzung E_{N}(i+1) in die der U_{N}(i) nachgeschaltete Teileinheit U_{N}(i+1) während eines regulären Betriebsmodus von U_{N}(i+1) eingespeist wird;
wobei die Edukt-Zusammensetzung E_{N}(1), die in die erste, am weitesten stromaufwärts gelegene Teileinheit U_{N}(1) eingespeist wird, Toluol als Z¹_{E,N}(1) umfasst, wobei noch bevorzugter j=1;
wobei das chemische Verfahren umfasst, während eines regulären Betriebsmodus einer Teileinheit U_{N}(i), einen Teil von P_{N}(i) in mindestens einer Speichervorrichtung D_{N}(m) wie in Anspruch 2 definiert zu speichern; wobei das chemische Verfahren umfasst, einen Teilprozess sp_{N}(i) in einer Teileinheit U_{N}(i) anzufahren, wobei das Anfahren das Zuführen mindestens eines Teils von P_{N}(i), der aus U_{N}(i) während des regulären Betriebsmodus von U_{N}(i) erhalten und in der mindestens einen Speichervorrichtung D_{N}(m) gespeichert wurde, als S_{N}(i) in U_{N}(i) umfasst, wobei vorzugsweise eine Produkt-Zusammensetzung P_{N}(i), die aus einer Teileinheit U_{N}(i) erhalten wird, eines oder mehrere von 2,4-Dinitrotoluol als Z¹_{P,N}(i), 2,6-Dinitrotoluol als Z²_{P,N}(i) und 2-Nitrotoluol als Z³_{P,N}(i) umfasst.

5. Das Verfahren nach Anspruch 4, ferner umfassend mindestens einen ersten zwischengeschalteten Behandlungs-teilprozess, wobei ein erster zwischengeschalteter Behandlungsteilprozess einen Phasentrennungsteilprozess, sp_{S}(i), umfasst, vorzugsweise daraus besteht, der in einer Teileinheit U_{S}(i) durchgeführt wird, wobei die aus U_{N}(i) erhaltene Produkt-Zusammensetzung P_{N}(i), bevor sie der Teileinheit U_{N}(i+1) zugeführt wird, als Edukt-Zusammensetzung E_{S}(i) einer Trennung unterzogen und während sp_{S}(i) in eine organische Phase und eine wässrige Phase getrennt wird, wobei die organische Phase, P_{S}(i), als Edukt-Zusammensetzung E_{N}(i+1) in die Teileinheit U_{N}(i+1) eingespeist wird, wobei die Teileinheit U_{S}(i), in der sp_{S}(i) durchgeführt wird, einen Phasenabscheider umfasst; wobei das chemische Verfahren umfasst, während eines regulären Betriebsmodus einer Teileinheit U_{S}(i), einen Teil von P_{S}(i) in mindestens einer Speichervorrichtung D_{S}(m) wie in Anspruch 2 definiert zu speichern;
wobei das chemische Verfahren umfasst, den Teilprozess sp_{S}(i) in der Teileinheit U_{S}(i) anzufahren,
wobei das Anfahren das Zuführen mindestens eines Teils von P_{S}(i), der aus U_{S}(i) während eines regulären Betriebsmodus von U_{S}(i) erhalten und in der mindestens einen Speichervorrichtung D_{S}(m) gespeichert wurde, als S_{S}(i) in U_{S}(i) umfasst; vorzugsweise umfassend das Anfahren des Teilprozesses sp_{N}(i) in einer Teileinheit U_{N}(i), wobei das Anfahren das Zuführen mindestens eines Teils von P_{S}(i), der aus U_{S}(i) erhalten und in der mindestens einen Speichervorrichtung D_{N}(m) gespeichert wurde, als S_{N}(i) in U_{N}(i) umfasst.

6. Das Verfahren nach Anspruch 5, wobei eine Produkt-Zusammensetzung P_{S}(i), die aus einer Teileinheit U_{S}(i) erhalten wird, eines oder mehrere von 2,4-Dinitrotoluol als Z¹_{P},_{S}(i), 2,6-Dinitrotoluol als Z²_{P,S}(i) und 2-Nitrotoluol als Z³_{P,S}(i) umfasst;
wobei die Produkt-Zusammensetzung P_{S}(i), die aus der am weitesten stromabwärts gelegenen Teileinheit U_{S}(i) erhalten wird,
vorzugsweise eines oder mehrere von 2,4-Dinitrotoluol als Z¹_{P},_{S}(i) und 2,6-Dinitrotoluol als Z²_{P,S}(i) umfasst, wobei vorzugsweise mindestens 75 mol-%, noch bevorzugter mindestens 80 mol-%, noch bevorzugter mindestens 85 mol-%, noch bevorzugter mindestens 90 mol-% aller in der Produkt-Zusammensetzung P_{S}(i), die aus der am weitesten stromabwärts gelegenen Teileinheit Us(i) erhalten wird, enthaltenen Z^{k}_{P},_{S}(i) aus einem oder mehreren von 2,4-Dinitrotoluol als Z¹_{P},_{S}(i) und 2,6-Dinitrotoluol als Z²_{P,S}(i) bestehen.

7. Das Verfahren nach Anspruch 5 oder 6, ferner umfassend mindestens einen zweiten zwischengeschalteten Behandlungs-
teilprozess, wobei ein zweiter zwischengeschalteter Behandlungsteilprozess einen Waschteilprozess, sp_{W}(i), umfasst, vorzugsweise daraus besteht, der in einer Teileinheit U_{W}(i) durchgeführt wird, wobei eine aus einer Teileinheit U_{N}(i) erhaltene Produkt-Zusammensetzung P_{N}(i) oder eine aus einer Teileinheit U_{S}(i) erhaltene Produkt-Zusammensetzung P_{S}(i) als Edukt-Zusammensetzung E_{W}(i) einem Waschvorgang unterzogen wird, wobei eine Produkt-Zusammensetzung P_{W}(i) erhalten wird, wobei die Teileinheit U_{W}(i), in der sp_{W}(i) durchgeführt wird,
eine Waschvorrichtung umfasst;
wobei das Verfahren vorzugsweise einen Teilprozess sp_{W}(1) umfasst, wobei sp_{W}(1) direkt stromabwärts des am weitesten stromabwärts gelegenen Teilprozesses sp_{N}(i), oder direkt stromabwärts des am weitesten stromabwärts gelegenen Teilprozesses sp_{S}(i), oder direkt stromabwärts des am weitesten stromabwärts gelegenen Teilprozesses sp_{N}(i) und direkt stromabwärts des am weitesten stromabwärts gelegenen
Teilprozesses sp_{S}(i), vorzugsweise direkt stromabwärts des am weitesten stromabwärts gelegenen Teilprozesses sp_{S}(i), durchgeführt wird;
wobei das Verfahren umfasst, während eines regulären Betriebsmodus einer Teileinheit U_{W}(i), einen Teil von P_{W}(i) in mindestens einer Speichervorrichtung D_{W}(m) wie in Anspruch 2 definiert zu speichern;
wobei das Verfahren umfasst, einen Teilprozess sp_{W}(i) in einer Teileinheit U_{W}(i) anzufahren, wobei das Anfahren das Zuführen mindestens eines Teils von P_{W}(i), der aus U_{W}(i) erhalten und in der mindestens einen Speichervorrichtung D_{W}(m) gespeichert wurde, als S_{W}(i) in U_{W}(i) umfasst.

8. Das Verfahren nach Anspruch 7, wobei eine Produkt-Zusammensetzung P_{W}(i), die aus einer Teileinheit U_{W}(i) erhalten wird, eines oder mehrere von 2,4-Dinitrotoluol als Z¹_{P},_{W}(i), 2,6-Dinitrotoluol als
Z²_{P},_{W}(i) und 2-Nitrotoluol als Z³_{P,W}(i) umfasst;
wobei die Produkt-Zusammensetzung P_{W}(i), die aus der am weitesten stromabwärts gelegenen Teileinheit U_{W}(i) erhalten wird, vorzugsweise eines oder mehrere von 2,4-Dinitrotoluol als Z¹_{P},_{W}(i) und 2,6-Dinitrotoluol als Z²_{P},_{W}(i) umfasst, wobei vorzugsweise mindestens 75 mol-%, noch bevorzugter mindestens 80 mol-%, noch bevorzugter mindestens 85 mol-%, noch bevorzugter mindestens 90 mol-% aller in der Produkt-Zusammensetzung P_{W}(i), die aus der am weitesten stromabwärts gelegenen Teileinheit U_{W}(i) erhalten wird, enthaltenen Z^{k}_{P},_{W}(i) aus einem oder mehreren von 2,4-Dinitrotoluol als Z¹_{P},_{W}(i) und 2,6-Dinitrotoluol als Z²_{P},_{W}(i) bestehen.

9. Das Verfahren nach einem der Ansprüche 1 bis 8, vorzugsweise nach einem der Ansprüche 4 bis 8, noch bevorzugter nach einem der Ansprüche 5 bis 8, noch bevorzugter nach Anspruch 7 oder 8, wobei ein oder mehrere
Teilprozesse sp(i) eine Hydrierung, sp_{H}(i), umfassen, wobei jede Teileinheit U_{H}(i), in der ein Teilprozess sp_{H}(i) durchgeführt wird, einen Hydrierungsreaktor umfasst, wobei eine Edukt-Zusammensetzung E_{H}(i), die in eine Teileinheit U_{H}(i) eingespeist wird, mindestens ein Hydrierungsmittel umfasst;
wobei das Verfahren vorzugsweise einen Teilprozess sp_{H}(1) umfasst;
wobei die mindestens eine aromatische Verbindung Z^{j}_{E.H}(i), die in E_{H}(i) enthalten ist, eines oder mehrere von 2,4-Dinitrotoluol als Z¹_{E,H}(i), 2,6-Dinitrotoluol als Z²_{E,H}(i), 2-Nitrotoluol als Z³_{E,H}(i) und 6-Nitrotoluol als Z⁴_{E,H}(i) ist;
wobei E_{H}(i) vorzugsweise eines oder mehrere von einer Produkt-Zusammensetzung P_{N}(i) wie in Anspruch 4 definiert, einer Produkt-Zusammensetzung P_{S}(i) wie in Anspruch 6 definiert und einer Produkt-Zusammensetzung P_{W}(i) wie in Anspruch 7 definiert, vorzugsweise eine Produkt-Zusammensetzung P_{W}(i) wie in Anspruch 7 definiert, umfasst; wobei das Verfahren umfasst, während eines regulären Betriebsmodus einer Teileinheit U_{H}(i),
einen Teil von P_{H}(i) in mindestens einer Speichervorrichtung D_{H}(m) wie in Anspruch 2 definiert zu speichern;
wobei das Verfahren umfasst, einen Teilprozess sp_{H}(i) in einer Teileinheit U_{H}(i) anzufahren,
wobei das Anfahren das Zuführen mindestens eines Teils von P_{H}(i), der aus U_{H}(i) erhalten und in der mindestens einen Speichervorrichtung D_{H}(m) gespeichert wurde, als S_{H}(i) in U_{H}(i) umfasst.

10. Das Verfahren nach Anspruch 9, wobei eine Produkt-Zusammensetzung P_{H}(i), die aus einer Teileinheit U_{H}(i) erhalten wird, eines oder mehrere von 2,4-Diaminotoluol als Z¹_{P,H}(i), 2,6-Diaminotoluol als Z²_{P,H}(i) und 2-Aminotoluol als Z³_{P,H}(i) umfasst.

11. Das Verfahren nach einem der Ansprüche 7 bis 10, ferner umfassend mindestens einen dritten zwischengeschalteten
Behandlungsteilprozess, wobei ein dritter zwischengeschalteter Behandlungsteilprozess einen Destillationsteilprozess, sp_{D}(i), umfasst, vorzugsweise daraus besteht, der in einer Teileinheit U_{D}(i) durchgeführt wird, wobei vorzugsweise eine aus einer Teileinheit U_{H}(i) erhaltene Produkt-Zusammensetzung P_{H}(i) als Edukt-Zusammensetzung E_{D}(i) einer Destillation unterzogen wird, wobei eine Produkt-Zusammensetzung P_{D}(i) erhalten wird, wobei die Teileinheit U_{D}(i), in der sp_{D}(i) durchgeführt wird, eine Destillationsvorrichtung umfasst und
wobei in U_{D}(i) E_{D}(i) in P_{D}(i) und mindestens eine weitere Destillationszusammensetzung C_{D}(i) getrennt wird;
wobei
- das Verfahren umfasst, während eines regulären Betriebsmodus einer Teileinheit U_{D}(i), einen Teil von P_{D}(i) in mindestens einer Speichervorrichtung D_{D,P}(m) wie in Anspruch 2 definiert zu speichern;
das Verfahren umfasst, einen Teilprozess sp_{D}(i) in einer Teileinheit U_{D}(i) anzufahren, wobei das Anfahren das Zuführen mindestens eines Teils von P_{D}(i), der aus U_{D}(i) erhalten und in der mindestens einen Speichervorrichtung D_{D,P}(m) gespeichert wurde, als S_{D}(i) in U_{D}(i) umfasst;
und/oder
- das Verfahren umfasst, während eines regulären Betriebsmodus einer Teileinheit U_{D}(i),
einen Teil von C_{D}(i) in mindestens einer Speichervorrichtung D_{D,C}(m) wie in Anspruch 2 definiert zu speichern;
das Verfahren umfasst, einen Teilprozess sp_{D}(i) in einer Teileinheit U_{D}(i) anzufahren, wobei das Anfahren das Zuführen mindestens eines Teils von C_{D}(i), der aus U_{D}(i) erhalten und in der mindestens einen Speichervorrichtung D_{D,C}(m) gespeichert wurde, als S_{D}(i) in U_{D}(i) umfasst.

12. Das Verfahren nach Anspruch 11, umfassend
- das Anfahren eines Teilprozesses sp_{H}(i) in einer Teileinheit U_{H}(i), wobei das Anfahren das Zuführen mindestens eines Teils von P_{D}(i), der aus U_{D}(i) erhalten und in mindestens einer Speichervorrichtung D_{D,P}(m) wie in Anspruch 11 definiert gespeichert wurde, als S_{H}(i) in U_{H}(i) umfasst;
und/oder
- das Anfahren eines Teilprozesses sp_{H}(i) in einer Teileinheit U_{H}(i), wobei das Anfahren das Zuführen mindestens eines Teils von U_{D}(i), der aus U_{D}(i) erhalten und in mindestens einer Speichervorrichtung D_{D,C}(m) wie in Anspruch 11 definiert gespeichert wurde, als S_{H}(i) in U_{H}(i) umfasst.

13. Das Verfahren nach Anspruch 11 oder 12, wobei eine Produkt-Zusammensetzung P_{D}(i), die aus einer Teileinheit U_{D}(i) erhalten wird, eines oder mehrere von 2,4-Diaminotoluol als Z¹_{P,D}(i), 2,6-Diaminotoluol als Z²_{P,D}(i) und 2-Aminotoluol als Z³_{P,D}(i) umfasst.

14. Das Verfahren nach einem der Ansprüche 11 bis 13, umfassend das Anfahren eines Teilprozesses sp_{D}(i) in einer Teileinheit U_{D}(i), wobei das Anfahren das Zuführen mindestens eines Teils von P_{H}(i), der aus U_{H}(i) erhalten und in der mindestens einen Speichervorrichtung D_{H}(m) wie in Anspruch 9 definiert gespeichert wurde, als S_{D}(i) in U_{D}(i) umfasst.

15. Eine chemische Einheit U zur Durchführung des Verfahrens nach einem der Ansprüche 1 bis 14, wobei die chemische Einheit U umfasst
(i.1) eine oder mehrere Teileinheiten U_{N}(i), vorzugsweise zwei Teileinheiten U_{N}(1) und U_{N}(2);
(i.2) eine oder mehrere Teileinheiten U_{S}(i), vorzugsweise zwei Teileinheiten U_{S}(1) und U_{S}(2);
(i.3) eine oder mehrere Teileinheiten U_{W}(i), vorzugsweise eine Teileinheit U_{W}(1);
(i.4) eine oder mehrere Speichervorrichtungen D_{N}(m), vorzugsweise zwei Speichervorrichtungen D_{N}(1) und D_{N}(2);
wobei U_{N}(1) stromaufwärts von U_{S}(1) angeordnet ist, U_{S}(1) stromaufwärts von U_{N}(2) angeordnet ist, U_{N}(2) stromaufwärts von U_{S}(2) angeordnet ist und U_{S}(2) stromaufwärts von U_{W}(1) angeordnet ist;
(ii.1) eine oder mehrere Teileinheiten U_{H}(i), vorzugsweise eine Teileinheit U_{H}(1);
(ii.2) eine oder mehrere Teileinheiten U_{D}(i), vorzugsweise eine Teileinheit U_{D}(1);
(ii.3) eine oder mehrere Speichervorrichtungen D_{H}(m), vorzugsweise eine Speichervorrichtung D_{H}(1);
(ii.4) eine oder mehrere Speichervorrichtungen D_{D}(m), vorzugsweise zwei Speichervorrichtungen D_{D,P}(1) und D_{D,C}(1);
wobei U_{H}(1) stromabwärts von U_{W}(1) und stromaufwärts von U_{D}(1) angeordnet ist;
(iii.1) eine oder mehrere Teileinheiten U_{P}(i), vorzugsweise eine Teileinheit U_{P}(1);
wobei U_{P}(1) stromabwärts von U_{D}(1) angeordnet ist.

## Revendications

1. Un procédé chimique pour préparer au moins un isocyanate aromatique, ledit procédé comprenant n sous-procédés chimiques sp(i), dans lequel un sous-procédé sp(i) est mis en œuvre dans une sous-unité de traitement chimique U(i), i=1... n, n≥1,
dans lequel, pendant un mode de fonctionnement normal d'une sous-unité U(i), le sous-procédé sp(i) mis en œuvre dans ladite sous-unité U(i) comprend l'alimentation d'une composition d'éducte E(i) comprenant au moins un composé aromatique Z^{j}_{E}(i), j≥1, dans ladite sous-unité U(i), le traitement de ladite composi-tion E(i) dans ladite sous-unité U(i) et l'obtention d'une composition de produit P(i) comprenant au moins un composé aromatique Z^{k}_{P}(i), k≥1,
dans lequel le procédé chimique comprend le démarrage dudit sous-procédé sp(i) dans ladite sous-unité U(i), ledit démarrage comprenant le traitement d'une composition chimique de démarrage S(i) dans ladite sous-unité U(i), dans lequel ledit S(i) ≠ E(i) et dans lequel ledit S(i) comprend au moins l'un des composés aromatiques Z^{k}_{P}(i).

2. Le procédé de la revendication 1, dans lequel, si n≥2, au moins 2, de préférence toutes les sous-unités U(i) font partie d'une unité chimique U, dans lequel U comprend en outre, en plus des n sous-unités de traitement chimique U(i), r dispositifs de stockage D(m), m=1... r, r≥1 ;
ledit procédé chimique comprenant en outre, pendant un mode de fonctionnement normal d'une sous-unité
U(i), le retrait de la composition de produit P(i) de ladite sous-unité U(i) et le transfert d'une por-tion de ladite composition de produit P(i) retirée de ladite sous-unité U(i) dans un dispositif de stockage D(m) ;
ledit procédé chimique comprenant en outre, pendant le démarrage du sous-procédé sp(i), l'alimentation d'au moins une portion de P(i), stockée dans D(m) pendant le mode de fonctionnement normal de la sous-unité U(i), en tant qu'au moins une portion de la composition chimique S(i) dans la sous-unité U(i).

3. Le procédé de la revendication 1 ou 2, dans lequel la sous-unité U(i) est sélectionnée dans le groupe constitué d'un réacteur chimique, d'une colonne de distillation, d'une colonne de rectification, d'un réservoir, d'un séparateur de phases, d'un dispositif de lavage, et d'une combinaison de deux ou plusieurs de ceux-ci, dans lequel au moins un sous-procédé sp(i) comprend une nitration et au moins un autre sous-procédé sp(i) comprend une hydrogénation; et dans lequel le au moins un isocyanate aromatique comprend de préférence un diisocyanate aromatique, plus préférablement un diisocyanate de toluène, plus préférablement un ou plusieurs parmi le diisocyanate de toluène 2,4 et le diisocyanate de toluène 2,6, ou un diisocyanate de méthylène diphényle, plus préférablement un ou plusieurs parmi le méthylène diphényle 2,2'-diisocyanate, le méthylène diphényle 2,4'-diisocyanate et le méthylène diphényle 4,4'-diisocyanate; dans lequel plus préférablement, le au moins un isocyanate aromatique comprend, de préférence est, un ou plusieurs parmi le diisocyanate de toluène 2,4 et le diisocyanate de toluène 2,6.

4. Le procédé de l'une quelconque des revendications 1 à 3, de préférence de la revendication 3, dans lequel un ou plusieurs sous-procédés sp(i) comprennent une nitration, sp_{N}(i), dans lequel chaque sous-unité U_{N}(i) dans laquelle un sous-procédé sp_{N}(i) est mis en œuvre comprend un réacteur de nitration, dans lequel une composition d'éducte E_{N}(i), alimentée dans une sous-unité U_{N}(i), comprend au moins un agent nitrant, de préférence l'a- cide nitrique, et comprend de préférence en outre au moins un agent déshydratant, de préférence l'acide sulfurique ;
dans lequel ledit procédé comprend de préférence deux ou plusieurs sous-procédés sp_{N}(i) qui sont mis en œuvre en séquence, dans lequel au moins une portion de la composition de produit P_{N}(i) ob-tenue d'une sous-unité U_{N}(i) est alimentée, optionnellement après un sous-procédé de traitement intermédiaire, en tant que composition d'éducte E_{N}(i+1) dans la sous-unité U_{N}(i+1) en aval de U_{N}(i) pendant un mode de fonctionnement normal de U_{N}(i+1) ;
dans lequel la composition d'éducte E_{N}(1) alimentée dans la première sous-unité la plus en amont U_{N}(1) comprend du toluène en tant que Z¹_{E,N}(1), dans lequel plus préférablement, j=1 ;
dans lequel ledit procédé chimique comprend, pendant un mode de fonctionnement normal d'une sous-unité U_{N}(i), le stockage d'une portion de P_{N}(i) dans au moins un dispositif de stockage D_{N}(m) tel que défini dans la revendication 2 ; dans lequel ledit procédé chimique comprend le démarrage d'un sous-procédé sp_{N}(i) dans une sous-unité U_{N}(i), ledit démarrage comprenant l'alimentation d'au moins une portion de P_{N}(i), obtenue de ladite U_{N}(i) pendant le mode de fonctionnement normal de U_{N}(i), et stockée dans le au moins un dispositif de stockage D_{N}(m), en tant que S_{N}(i) dans U_{N}(i), dans lequel de préférence, une composition de produit P_{N}(i), obtenue d'une sous-unité U_{N}(i), comprend un ou plusieurs parmi le 2,4-dinitrotoluène en tant que Z¹_{P,N}(i), le 2,6-dinitrotoluène en tant que Z²_{P,N}(i), et le 2-nitrotoluène en tant que Z³_{P,N}(i).

5. Le procédé de la revendication 4, comprenant en outre au moins un premier sous-procédé de traitement intermédiaire, dans lequel un premier sous-procédé de traitement intermédiaire comprend, de préférence consiste en un sous-procédé de séparation de phases, sp_{S}(i), qui est mis en œuvre dans une sous-unité U_{S}(i), dans lequel la composition de produit P_{N}(i) obtenue de U_{N}(i), avant d'être alimentée dans la sous-unité U_{N}(i+1), est soumise en tant que composition d'éducte E_{S}(i) à une séparation et est séparée pendant sp_{S}(i) en une phase organique et une phase aqueuse, dans lequel la phase organique, P_{S}(i), est alimentée en tant que composition d'éducte E_{N}(i+1) dans la sous-unité U_{N}(i+1), dans lequel la sous-unité U_{S}(i) dans laquelle sp_{S}(i) est mis en œuvre comprend un séparateur de phases ;
dans lequel ledit procédé chimique comprend, pendant un mode de fonctionnement normal d'une sous-unité U_{S}(i), le stockage d'une portion de P_{S}(i) dans au moins un dispositif de stockage D_{S}(m) tel que défini dans la revendication 2 ;
dans lequel ledit procédé chimique comprend le démarrage du sous-procédé sp_{S}(i) dans la sous-unité U_{S}(i), ledit démarrage comprenant l'alimentation d'au moins une portion de P_{S}(i), obtenue de ladite U_{S}(i) pen-dant un mode de fonctionnement normal de U_{S}(i) et stockée dans le au moins un dispositif de stockage D_{S}(m), en tant que S_{S}(i) dans U_{S}(i) ; comprenant de préférence le démarrage du sous-procédé sp_{N}(i) dans une sous-unité U_{N}(i), ledit démarrage comprenant l'alimentation d'au moins une portion de P_{S}(i), obtenue de ladite U_{S}(i) et stockée dans le au moins un dispositif de stockage D_{N}(m), en tant que S_{N}(i) dans U_{N}(i).

6. Le procédé de la revendication 5, dans lequel une composition de produit P_{S}(i), obtenue d'une sous-unité U_{S}(i), comprend un ou plusieurs parmi le 2,4-dinitrotoluène en tant que Z¹_{P},_{S}(i), le 2,6-dinitrotoluène en tant que Z²_{P},_{S}(i), et le 2-nitrotoluène en tant que Z³_{P},_{S}(i) ; dans lequel la composition de produit P_{S}(i), obtenue de la sous-unité la plus en aval U_{S}(i), comprend de préférence un ou plusieurs parmi le 2,4-dinitrotoluène en tant que Z¹_{P},_{S}(i) et le 2,6-dinitrotoluène en tant que Z²_{P},_{S}(i), dans lequel de préférence au moins 75 mol-%, plus préférablement au moins 80 mol-%, plus préférablement au moins 85 mol-%, plus préférablement au moins 90 mol-% de tous les Z^{k}_{P},_{S}(i) compris dans la composition de produit P_{S}(i) obtenue de la sous-unité la plus en aval Us(i) sont constitués d'un ou plusieurs parmi le 2,4-dinitrotoluène en tant que Z¹_{P},_{S}(i) et le 2,6-dinitrotoluène en tant que Z²_{P},_{S}(i).

7. Le procédé de la revendication 5 ou 6, comprenant en outre au moins un deuxième sous-procédé de traitement intermédiaire, dans lequel un deuxième sous-procédé de traitement intermédiaire comprend, de préférence consiste en un sous-procédé de lavage, sp_{W}(i), qui est mis en œuvre dans une sous-unité U_{W}(i), dans lequel une composition de produit P_{N}(i) obtenue d'une sous-unité U_{N}(i), ou une composition de produit P_{S}(i) obtenue d'une sous-unité U_{S}(i), est soumise en tant que composition d'éducte E_{W}(i) à un lavage, ob-tenant une composition de produit P_{W}(i), dans lequel la sous-unité U_{W}(i) dans laquelle sp_{W}(i) est mis en œuvre comprend un dispositif de lavage ;
dans lequel ledit procédé comprend de préférence un sous-procédé sp_{W}(1), dans lequel sp_{W}(1) est mis en œuvre directement en aval du sous-procédé le plus en aval sp_{N}(i), ou directement en aval du sous-procédé le plus en aval sp_{S}(i), ou directement en aval du sous-procédé le plus en aval sp_{N}(i) et directement en aval du sous-procédé le plus en aval sp_{S}(i), de préférence directement en aval du sous-procédé le plus en aval sp_{S}(i) ;
dans lequel ledit procédé comprend, pendant un mode de fonctionnement normal d'une sous-unité U_{W}(i), le stockage d'une portion de P_{W}(i) dans au moins un dispositif de stockage D_{W}(m) tel que défini dans la revendication 2 ;
dans lequel ledit procédé comprend le démarrage d'un sous-procédé sp_{W}(i) dans une sous-unité U_{W}(i), ledit démarrage comprenant l'alimentation d'au moins une portion de P_{W}(i), obtenue de ladite U_{W}(i) et stockée dans le au moins un dispositif de stockage D_{W}(m), en tant que S_{W}(i) dans U_{W}(i).

8. Le procédé de la revendication 7, dans lequel une composition de produit P_{W}(i), obtenue d'une sous-unité U_{W}(i), comprend un ou plusieurs parmi le 2,4-dinitrotoluène en tant que Z¹_{P},_{W}(i), le 2,6-dinitrotoluène en tant que Z²_{P},_{W}(i), et le 2-nitrotoluène en tant que Z³_{P,W}(i) ; dans lequel la composition de produit P_{W}(i), obtenue de la sous-unité la plus en aval U_{W}(i), comprend de préférence un ou plusieurs parmi le 2,4-dinitrotoluène en tant que Z¹_{P},_{W}(i) et le 2,6-dinitrotoluène en tant que Z²_{P},_{W}(i), dans lequel de préférence au moins 75 mol-%, plus préférablement au moins 80 mol-%, plus préférablement au moins 85 mol-%, plus préférablement au moins 90 mol-% de tous les Z^{k}_{P},_{W}(i) compris dans la composition de produit P_{W}(i) obtenue de la sous-unité la plus en aval U_{W}(i) sont constitués d'un ou plusieurs parmi le 2,4-dinitrotoluène en tant que Z¹_{P},_{W}(i) et le 2,6-dinitrotoluène en tant que Z²_{P},_{W}(i).

9. Le procédé de l'une quelconque des revendications 1 à 8, de préférence de l'une quelconque des revendications 4 à 8, plus préférablement de l'une quelconque des revendications 5 à 8, plus préférablement de la revendication 7 ou 8, dans lequel un ou plusieurs sous-procédés sp(i) comprennent une hydrogénation, sp_{H}(i), dans lequel chaque sous-unité U_{H}(i) dans laquelle un sous-procédé sp_{H}(i) est mis en œuvre comprend un réacteur d'hydrogénation, dans lequel une composition d'éducte E_{H}(i), alimentée dans une sous-unité U_{H}(i), comprend au moins un agent d'hydrogénation ;
dans lequel ledit procédé comprend de préférence un sous-procédé sp_{H}(1) ;
dans lequel le au moins un composé aromatique Z^{j}_{E.H}(i) compris dans E_{H}(i) est un ou plusieurs parmi le 2,4-dinitrotoluène en tant que Z¹_{E,H}(i), le 2,6-dinitrotoluène en tant que Z²_{E,H}(i), le 2-nitrotoluène en tant que Z³_{E,H}(i) et le 6-nitrotoluène en tant que Z⁴_{E,H}(i) ;
dans lequel E_{H}(i) comprend de préférence un ou plusieurs parmi une composition de produit P_{N}(i) telle que définie dans la revendication 4, une composition de produit P_{S}(i) telle que définie dans la revendication 6, et une composition de produit P_{W}(i) telle que définie dans la revendication 7, de préférence une composition de produit P_{W}(i) telle que définie dans la revendication 7 ; dans lequel ledit procédé comprend, pendant un mode de fonctionnement normal d'une sous-unité U_{H}(i), le stoc kage d'une portion de P_{H}(i) dans au moins un dispositif de stockage D_{H}(m) tel que défini dans la revendication 2 ;
dans lequel ledit procédé comprend le démarrage d'un sous-procédé sp_{H}(i) dans une sous-unité U_{H}(i), ledit démarrage comprenant l'alimentation d'au moins une portion de P_{H}(i), obtenue de ladite U_{H}(i) et stockée dans le au moins un dispositif de stockage D_{H}(m), en tant que S_{H}(i) dans U_{H}(i).

10. Le procédé de la revendication 9, dans lequel une composition de produit P_{H}(i), obtenue d'une sous-unité U_{H}(i), comprend un ou plusieurs parmi le 2,4-diaminotoluène en tant que Z¹_{P,H}(i), le 2,6-diaminotoluène en tant que Z²_{P,H}(i), et le 2-aminotoluène en tant que Z³_{P,H}(i).

11. Le procédé de l'une quelconque des revendications 7 à 10, comprenant en outre au moins un troisième sous-procédé de traitement intermédiaire, dans lequel un troisième sous-procédé de traitement intermédiaire comprend, de préférence consiste en un sous-procédé de distillation, sp_{D}(i), qui est mis en œuvre dans une sous-unité U_{D}(i), dans lequel de préférence, une composition de produit P_{H}(i) obtenue d'une sous-unité U_{H}(i) est soumise en tant que composition d'éducte E_{D}(i) à une distillation, obtenant une composition de produit P_{D}(i), dans lequel la sous-unité U_{D}(i) dans laquelle sp_{D}(i) est mis en œuvre comprend un dispositif de distillation et
dans lequel dans U_{D}(i), E_{D}(i) est séparée en P_{D}(i) et au moins une autre composition de distillation C_{D}(i) ;
dans lequel
- ledit procédé comprend, pendant un mode de fonctionnement normal d'une sous-unité U_{D}(i), le stockage d'une portion de P_{D}(i) dans au moins un dispositif de stockage D_{D,P}(m) tel que défini dans la revendication 2 ;
ledit procédé comprenant le démarrage d'un sous-procédé sp_{D}(i) dans une sous-unité U_{D}(i), ledit démarrage comprenant l'alimentation d'au moins une portion de P_{D}(i), obtenue de ladite U_{D}(i) et stockée dans le au moins un dispositif de stockage D_{D,P}(m), en tant que S_{D}(i) dans U_{D}(i) ;
et/ou
- ledit procédé comprend, pendant un mode de fonctionnement normal d'une sous-unité U_{D}(i), le stockage d'une portion de C_{D}(i) dans au moins un dispositif de stockage D_{D,C}(m) tel que défini dans la revendication 2 ;
ledit procédé comprenant le démarrage d'un sous-procédé sp_{D}(i) dans une sous-unité U_{D}(i), ledit démarrage comprenant l'alimentation d'au moins une portion de C_{D}(i), obtenue de ladite U_{D}(i) et stockée dans le au moins un dispositif de stockage D_{D,C}(m), en tant que S_{D}(i) dans U_{D}(i).

12. Le procédé de la revendication 11, comprenant
- le démarrage d'un sous-procédé sp_{H}(i) dans une sous-unité U_{H}(i), ledit démarrage comprenant l'alimen-tation d'au moins une portion de P_{D}(i), obtenue de U_{D}(i) et stockée dans au moins un dispositif de stockage D_{D,P}(m) tel que défini dans la revendication 11, en tant que S_{H}(i) dans U_{H}(i) ;
et/ou
le démarrage d'un sous-procédé sp_{H}(i) dans une sous-unité U_{H}(i), ledit démarrage comprenant l'alimen-tation d'au moins une portion de U_{D}(i), obtenue de U_{D}(i) et stockée dans au moins un dispositif de stockage D_{D,C}(m) tel que défini dans la revendication 11, en tant que S_{H}(i) dans U_{H}(i).

13. Le procédé de la revendication 11 ou 12, dans lequel une composition de produit P_{D}(i), obtenue d'une sous-unité U_{D}(i), comprend un ou plusieurs parmi le 2,4-diaminotoluène en tant que Z¹_{P,D}(i), le 2,6-diaminotoluène en tant que Z²_{P,D}(i), et le 2-aminotoluène en tant que Z³_{P,D}(i).

14. Le procédé de l'une quelconque des revendications 11 à 13, comprenant le démarrage d'un sous-procédé sp_{D}(i) dans une sous-unité U_{D}(i), ledit démarrage comprenant l'alimentation d'au moins une portion de P_{H}(i), obtenue de U_{H}(i) et stockée dans le au moins un dispositif de stockage D_{H}(m) tel que défini dans la revendication 9, en tant que S_{D}(i) dans U_{D}(i).

15. Une unité chimique U pour mettre en œuvre le procédé selon l'une quelconque des revendications 1 à 14, dans lequel ladite unité chimique U comprend
(i.1) une ou plusieurs sous-unités U_{N}(i), de préférence deux sous-unités U_{N}(1) et U_{N}(2) ;
(i.2) une ou plusieurs sous-unités U_{S}(i), de préférence deux sous-unités U_{S}(1) et U_{S}(2) ;
(i.3) une ou plusieurs sous-unités U_{W}(i), de préférence une sous-unité U_{W}(1) ;
(i.4) un ou plusieurs dispositifs de stockage D_{N}(m), de préférence deux dispositifs de stockage D_{N}(1) et D_{N}(2) ; dans lequel U_{N}(1) est disposé en amont de U_{S}(1), U_{S}(1) est disposé en amont de U_{N}(2), U_{N}(2) est disposé en amont de U_{S}(2), et U_{S}(2) est disposé en amont de U_{W}(1) ;
(ii.1) une ou plusieurs sous-unités U_{H}(i), de préférence une sous-unité U_{H}(1) ;
(ii.2) une ou plusieurs sous-unités U_{D}(i), de préférence une sous-unité U_{D}(1) ;
(ii.3) un ou plusieurs dispositifs de stockage D_{H}(m), de préférence un dispositif de stockage D_{H}(1) ;
(ii.4) un ou plusieurs dispositifs de stockage D_{D}(m), de préférence deux dispositifs de stockage D_{D,P}(1) et D_{D,C}(1) ;
dans lequel U_{H}(1) est disposé en aval de U_{W}(1) et en amont de U_{D}(1) ;
(iii.1) une ou plusieurs sous-unités U_{P}(i), de préférence une sous-unité U_{P}(1) ;
dans lequel U_{P}(1) est disposé en aval de U_{D}(1).
